# EUROPEAN PATENT APPLICATION

(11) **EP 1 643 352 A1**
(43) Date of publication of application: **05.04.2006**
(21) Application number: 04746473.0
(22) Date of filing: 25.06.2004
(51) Int. Cl.: G06F 3/06, G06F 12/00, G06F 17/30

(54) **DATABASE SYSTEM**

(30) Priority: 02.07.2003 JP 2003270402
(71) Applicant: Yamatake, Satoshi, Chofu-shi, Tokyo 182007 (JP)
(72) Inventor: Yamatake, Satoshi, Chofu-shi, Tokyo 182007 (JP)
(74) Representative: Betten & Resch
(86) International application number: PCT/JP2004/009004
(87) International publication number: WO 2005/003951

(57) **Abstract**

It is an object of the present invention to provide a database system capable of being rapidly accessed on the network without using a different media from the media stored primarily and capable of safely and stably backing up data without through man power. The database system comprising: a plurality of storage units 1 for storing image data; a control unit 2 for controlling the storage units 1; and a database server 3 storing attribute information including key information associated with the image data. The control unit includes operating state inspection means and redundancy storing means. The operating state inspection means executes an operating state inspection for investigating the data of residual capacity and the absence or presence of abnormalities to the storage unit at a predetermined timing, and storing the result on a table showing an operating state. The redundancy storing means selects and stores the number of redundancy of data storage locations for data storage request from the storage units identified based on the table showing the operating state and normally operated.

## Description

### TITLE OF THE INVENTION

The present invention relates to an image database system for storing a large quantity of medical image information photographed by a modality device such as a CT device and an MRI device as electronic information, and for contributing to diagnosis. In addition, the present invention relates to a database system such as a document database system stored so that a large quantity of document files can be retrieved as the electronic information.

### BACKGROUND

Referring to a conventional medical image database system, image data temporarily stored on a hard disk unit has been moved to a removable media such as a DVD and a MO so as to permanently store image data as disclosed in Japanese Unexamined Patent Publication No. 111987 (2002). The above work for moving medical record to the removable media is done manually, and has lead to an increase in cost and the disappearance of the image data due to human errors. Thereby, there is proposed an image control system, comprising a network connection means for transferring and receiving a image file through a network, a hard disk as first image storage means for temporarily saving the image file, a removable media as second image storage means for saving the image file permanently, and control means for controlling the storage process of the image file received through the network. The control means responds to the reception of the image file which record indication is given, and saves the image file in the first or second image storage means indicated.

### DISCLOSURE OF THE INVENTION

### PROBLEMS TO BE SOLVED BY THE INVENTION

However, referring to the conventional image control system described above, a large quantity of image data is permanently saved in a different media from a media for primary storage. Thereby, a media for a large quantity of permanent storage is required, and the storage place and control of the media are required. In addition, there is an essential problem that once the image data is saved on the DVD and the MO for permanent storage or the like, it cannot be rapidly accessed on the network when required.

The present invention has been accomplished in view of the foregoing and other problems in prior art. Accordingly, it is an object of the present invention to provide a database system capable of being rapidly accessed on the network without using the different media from the media stored primarily, and capable of taking a back-up data safely and stably without being handled manually.

### MEANS FOR SOLVING THE PROBLEMS

An image database system according to the present invention so as to attain the object, comprising:
a plurality of storage units for storing a document or image data;
a control unit for controlling the storage unit; and
a database server storing attribute information including key information associated with the document or the image data,
wherein the control unit includes:
operating state inspection means for executing an operating state inspection for investigating the data of residual capacity and the absence or presence of abnormalities to the storage unit at a predetermined timing, and storing the result on a table showing an operating state; and
redundancy storing means for selecting and storing the number of redundancy of data storage locations for data storage request from the storage units identified based on the table showing the operating state and normally operated.

According to the configuration described above, the normal or unusual operating state of the storage unit is regularly or irregularly searched by the control unit at a prescribed time, and the result is recorded on the table showing the operating state. Therefore, the data is safely and certainly stored in the normally operated storage unit. In addition, since the data stored is made redundant by the redundancy storing means and at least the data of only the same number of redundancy is stored, it is not necessary to back up the data in the other media for the disappearance of the data or the like. Even the data for back up can be always and rapidly accessed in a seek time within a predetermined time by the above constitution.

The database system according to claim 1, wherein the redundancy storing means investigates disk residual capacity in a drive unit based on the table showing the operating state, and selects the number of redundancy of drives of which the disk residual capacity is large to the different storage unit.

According to the configuration described above, the safety of the system can be improved by storing a series of data in the different storage unit by making redundant, and storing the data, and further a series of data can be stored in the same drive of the storage unit selected. Thereby, the controlling the access to the data can be easily performed. In addition, the concentration of access to the specific drive of the specific storage unit can be avoided, and the load can be distributed. Therefore, the physical and mechanical attrition of the drive is also distributed, and the reliability and stability as the system can be secured.

The database system according to claim 1, wherein the redundancy storing means investigates disk residual capacity in the storage unit based on the table showing the operating state, selects the number of redundancy of drives of which the disk residual capacity is large, and selects the drive of which the disk residual capacity is large from the selected storage units.

According to the configuration described above, the safety of the system can be improved by storing a series of data in the different storage unit by making redundant, and storing the data. Further, a series of data can be stored in the unit selected. Thereby, the access control of data can be easily performed. In addition, the concentration of access to the specific drive of the specific storage unit can be avoided, and the load can be distributed. Therefore, the physical and mechanical attrition of the storage unit is also distributed, and the reliability and stability as the system can be secured.

The database system according to claim 1, wherein the redundancy storing means selects a plurality of storage units having residual capacity of a predetermined capacity or more as a candidate based on the table showing the operating state, and selects the storage unit as an object based on the random number generated by random number generation means.

According to the configuration described above, the redundancy storing means randomly selects the specific storage unit based on the random number from the storage units 1 in which the disk residual capacity is large. Therefore, the concentration of access to the drives of the storage units 1 in which the disk residual capacity is large can be avoided can be prevented.

The database system according to any one of claims 2 to 4, wherein the redundancy storing means selects the storage units of the number of redundancy mounted on different frames based on the table showing the operating state.

According to the configuration described above, the redundancy storing means can suppress the influence caused by disasters such as fire, earthquake and flood by setting each frame in a different place and building.

The database system according to any one of claims 1 to 5, wherein the data storage location is stored as the attribute information in the database server.

The database system according to any one of claims 1 to 6, wherein the control unit acquires the data storage locations of other storage units in which the same data as the data stored in the storage unit judged to be unusual by the operating state inspection means is stored based on the attribute information stored in the database server, selects the data stored in the data storage location from the storage units identified based on the table showing an operating state and normally operated except the storage unit in which the same data is stored, and reproduces.

The database system according to any one of claims 1 to 6, wherein the control unit acquires the data storage location of the other storage units in which the same data as the data stored in the drive judged to be unusual by the operating state inspection means is stored based on the attribute information stored in the database server, selects and reproduces the data stored in the data storage location from the storage units identified based on the table showing the operating state and normally operated except the drive in which the same data is stored

That is, the data storage location of the other storage unit in which the same data as the data stored in the storage unit or drive being in the abnormal condition is stored can be grasped based on the attribute information stored in the database server, and the data which should be restored can be secured. The same data as the secured data is reproduced in any drive of other storage unit operating normally, or the normally operated storage units which are identified based on the table showing the operating state are selected and reproduced in the drive other than in which the same data is stored. Thereby, the existance of at least a pair of the same data in the system can be assured.

### ADVANTAGEOUS EFFECT OF THE INVENTION

As described above, the present invention can provide the database system capable of being rapidly accessed on the network without using a different media from the media stored primarily and capable of safely and stably backing up the data without through man power.

### BEST MODE FOR CARRYING OUT THE INVENTION

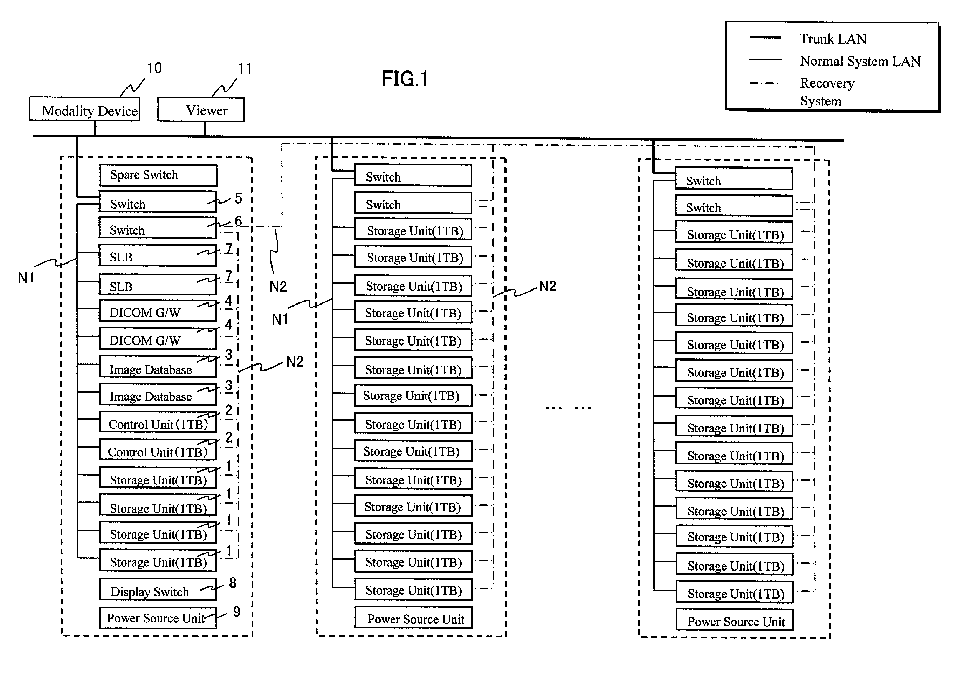

Referring to the drawings, the image database system according to the present invention will be described below. As shown in Fig. 1, the image database system comprises a basic frame (basic minimum tower) and a plurality of extended frames (expanded tower).

The basic frame is constituted by subjecting a storage unit group, a control unit group, an image database server group and a DICOM gateway group with each other to network connection through switches 5 and 6. The storage unit group contains four storage units 1 for storing medical image data. The control unit group contains two control units 2 for controlling each storage unit 1. The image database server group contains two image database servers 3 for storing attribute information including key information associated with the medical image data stored in each storage unit 1 and for applying relay processing of the medical image data between an externally connected viewer 11 and the image database server. The DICOM gateway group contains two DICOM gateways 4 for applying relay processing of the medical image data via a DICOM protocol between a plurality of modality devices 10 externally connected to the image database server 3 and the DICOM gateways. Two load balancers 7 for executing load distribution control of every group is provided for every group based on header information of a request through the network, and a power source unit 8 and a display switch 9 are provided. Herein, the number of units or servers should be a mere exemplary, and the invention is not limited thereto.

The extended frame comprises a power source unit, a plurality of storage units and switches. The extended frame can be extended so that image data with a high capacity can be stored.

The storage units 1, the control units 2, the image database servers 3 and the DICOM gateways 4 are subjected with each other to network connection by a first network N1 through a first switch 5 for processing a job related to an external request from the viewer 11 or the modality device 10, and a second network N2 through a second switch 6 for processing a job related to an internal request. In the networks N1 and N2, a physical layer and data link layer of the network are constituted by Ethernet (registered trademark) of 1000 BASE-T in an OSI reference standard model, and a transport layer and a network layer are constituted by a TCP/IP. High order layers of a session layer or more are operated by a DICOM protocol which is an image diagnostic standard in a medical field, or another local high order protocol.

Two load balancers 7 are connected to each other by a local communication line (RS232-C), and one load balancer is usually operated. For example, load distribution control is executed so that the load of each component may become equivalent by a round-robin system if there is no difference in the performance of the component of each group, a weighted round-robin system if there is a difference in the performance, and a response time algorithm or the like. When a failure is detected through the local communication line, a spare load balancer is operated. Herein, it is also possible to adopt the communication line by another telecommunication standard other than RS232-C as the local communication line. In addition, the local communication may be performed through the first or second network.

The operation of the system will be explained below by using the response operation or the like to the image storage request, the image read request from the modality device, and the image inspection request from the viewer as an example.

The modality device 10 transfers the image storage request via the DICOM protocol at a virtual IP address (VIP) assigned to the group constituted by two DICOM gateways 4, and starts an association. The load balancer 7 operates normally, and selects the DICOM gateway 4 having the least load. The load balancer 7 intercepts a packet having the virtual IP address as a destination address, and rewrites it to the real IP address of the DICOM gateway 4 selected. The image storage request is received by the selected DICOM gateway 4 according to the above process. The communication of returning packet is established by operating reversely. Herein, the load of the DICOM gateway 4 is distributed in one association by the load balancer 7.

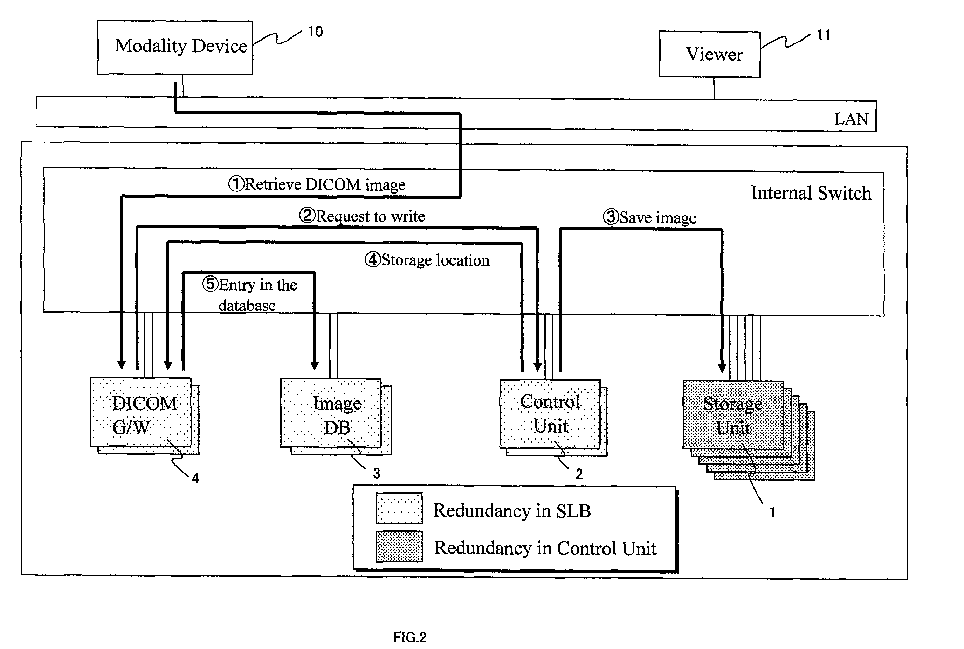

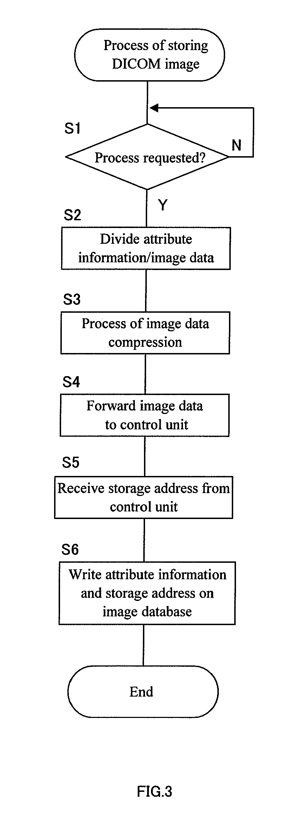

As shown in Figs. 2 and 3, the DICOM gateway 4 requested divides the received image information into the attribute information and image information containing an inspection example UID, the name of a patient, a patient ID, an acceptance number, inspection date, inspection time, and the date of birth of a patient. The inspection example UID consists of a distribution source fronting code, a product specific number and a serial number or the like. The divided image data are compressed into a plurality of images each of having different compression ratios by the DICOM gateway 4 and reversible compression process at a predetermined algorithm is given to each of those images. The compressed images are transferred to the control unit 2. Herein, though four kinds of images (an original image having 512 x 512 pixels, a 1/4 image having 256 x 256 pixels, a 1/16 image having 128 x 128 pixels, and a 1/64 image having 64 x 64 pixels used as a thumbnail) are generated and stored, the compressed images are not limited thereto. Though PNG (Portable Network Graphics) is used, the other reversible compression algorithm may be used.

The control unit 2 receives the image data transferred. The control unit 2 stores the image data with the algorithm to be described below, and replies the storage address to the DICOM gateway 4. The DICOM gateway 4 transfers the attribute information and the storage address of the image data replied from the control unit 2 to the image database server 3, and directs the update processing of data.

The control unit 2 and the image database server 3 involved in the above operation are communicated through the first network N1 in the same manner as the DICOM gateway 4, and the load is distributed by the load balancer 7. Though the load of each operation to be explained below is fundamentally distributed by the load balancer 7 as described above, the explanation of the operation of the load balancer is omitted afterward.

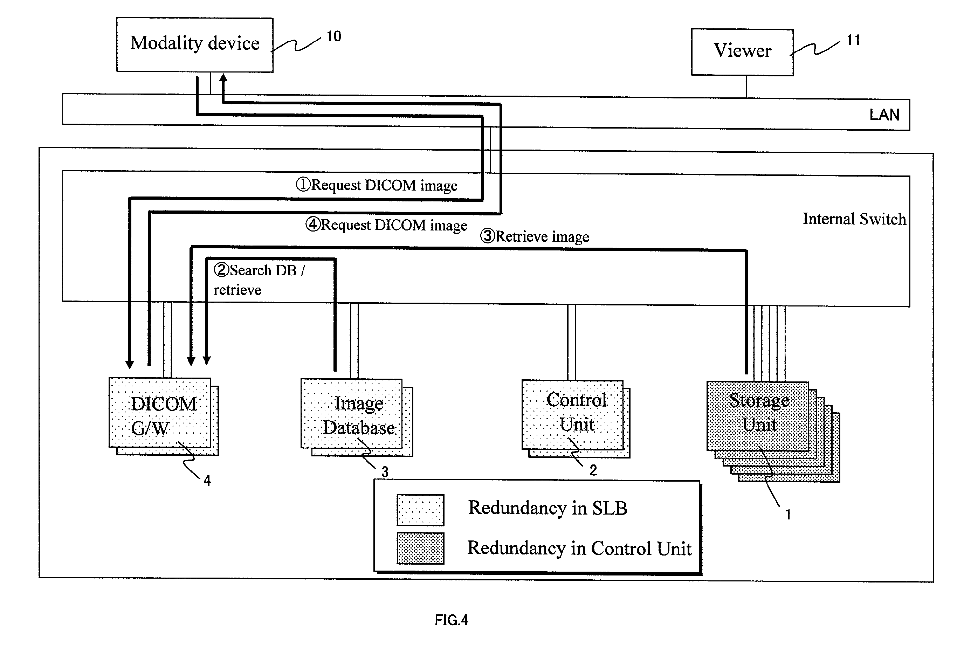

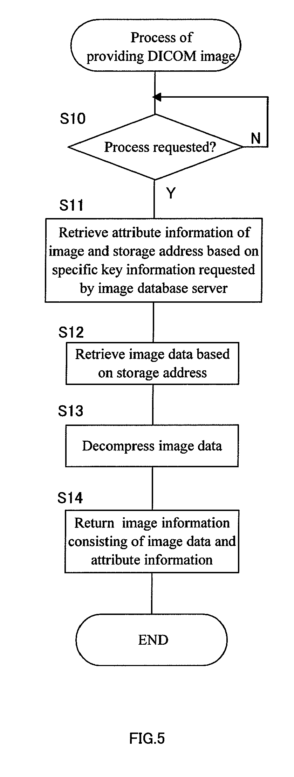

Next, when the DICOM gateway 4 receives an image providing request from the modality device 10, as shown in Figs. 4 and 5, the DICOM gateway 4 retrieves the attribute information and the storage address from the image database server 3 based on any key information of the attribute information specifying a request image. The DICOM gateway 4 retrieves the compressed image reversibly compressed from the storage unit 1 based on the storage address, decompresses the compressed image, and replies with the attribute information to the modality device 10 via the DICOM protocol.

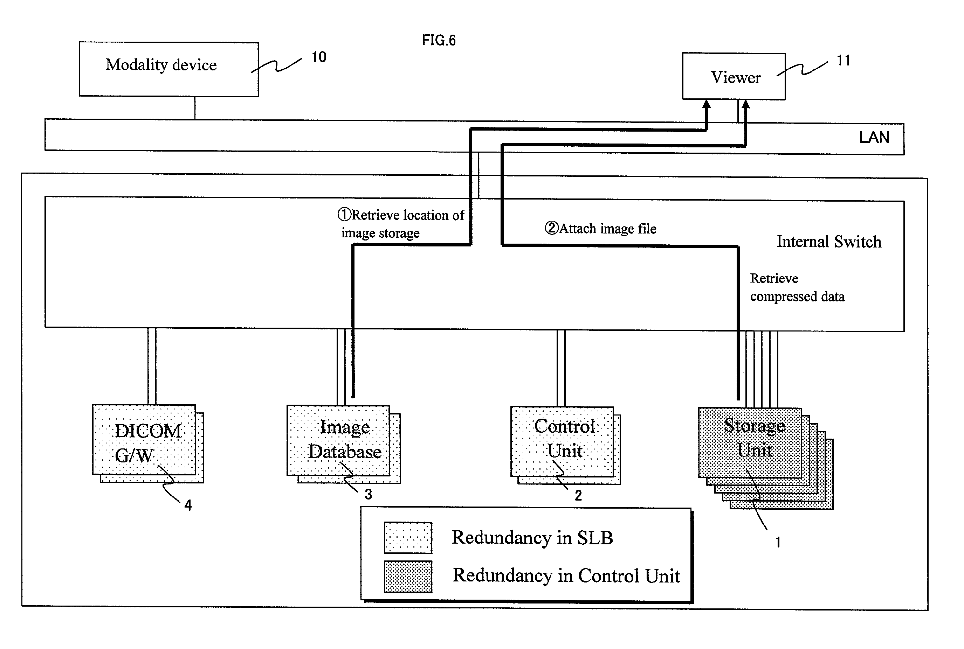

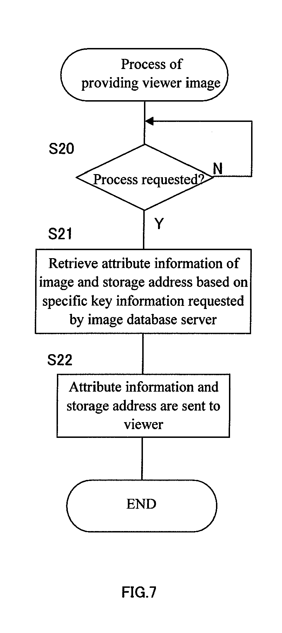

As shown in Figs. 6 and 7, the DICOM gateway 4 retrieves the attribute information and the storage address from the image database server 3 based on any key information of the attribute information specifying the requested image according to the inspection request of the image data from the viewer 11 to the image database server 3, and replies to the viewer 11. Then, the viewer 11 accesses the storage unit 1 based on the storage address received, and retrieves a desired image data. Herein, if the viewer 11 is the DICOM viewer, the viewer 11 is operated through the above DICOM gateway 4. However, if the DICOM protocol is not used, and the local protocol by TCP/IP is supported, any high order protocol can be used.

The control unit 2 is provided with operating state inspection means and failure diagnosis means. The operating state inspection means performs an operating state inspection in order through the second network N2 to each storage unit 1 without through the load balancer 7 when a power source is turned on (The load of the external access to the control unit 2 is distributed by the load balancer 7.). Then, the operating state inspection means updates and stores the result in the table showing the operating state of the image database server 3. The failure diagnosis means performs a failure diagnosis of the other control unit based on the update history of the table showing the operating state data due to the other control unit 2.

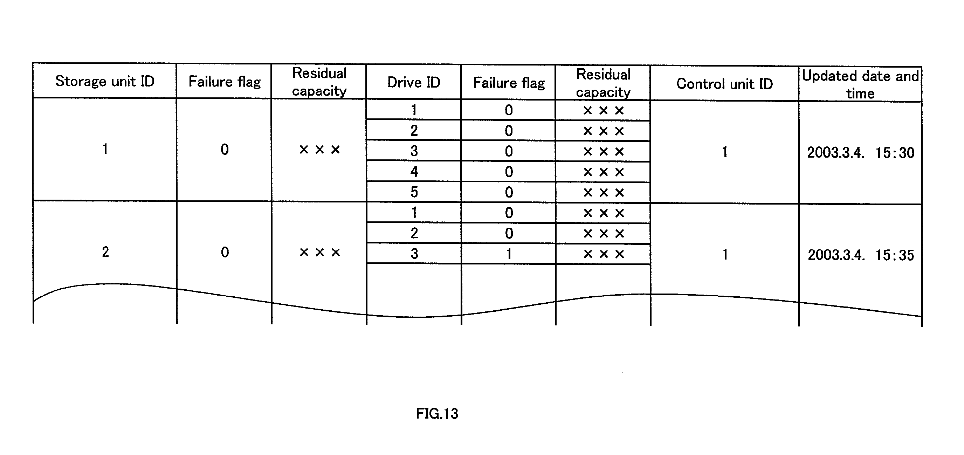

To be detailed below, the operating state inspection means check and see the attachment status of a storage unit in order in a frame unit, whether the unit is removed or attached, in the storage unit 1 which consists of several hard disks and is loaded on the frame, and in a case which the unit is attached, the disk residual capacity for every drive and the total residual capacity of the unit are given. In addition, the operating state inspection means performs the writing and reading of any file to each drive, and investigates the disk obstacle of each drive. The operating state inspection means updates and stores the result at the table showing the operating state of the database server 3 shown in Fig. 13. Though not apparently written in Fig. 13, the data can be controlled in the frame unit by adding a frame ID to the high order data of the storage unit ID.

The control unit 2 is provided with the redundancy storing means for selecting data storage locations of the number of redundancy from the storage units which are identified based on the table showing the operating state and are normally operated to the data storage request to the storage unit 1, and for and storing the data.

To be detailed below, the redundancy storing means investigates the disk residual capacity per drive based on the table showing the operating state, selects the drives of the number of redundancy in which the disk residual capacity is large to a different storage unit, and stores. Herein, the number of redundancy is set to 2. Therefore, the same data will certainly exist in the different storage unit 1 within the system. (Herein, it is needless to say that the number of redundancy may be set to 2 or more.)

Another method can investigate the disk residual capacity per the storage unit 1, and select two storage units 1 in which the disk residual capacity is large. In addition, the method can select a drive in which the disk residual capacity is large from the storage units 1 selected, and stores the data. The storage address of the data is replied to the transferring origin of a storage request. A reply to the storage request of the above image data is sent to the DICOM gateway 4, and is stored in the database server 3 with the attribute information of the image data or as the attribute information by the DICOM gateway 4.

According to another method, the redundancy storing means may select a plurality of storage units 1 having the residual capacity of a predetermined capacity or more as a candidate based on the table showing the operating state, and select two storage units 1 as an object based on a random number generated by the random number generation means. For example, the storage unit of more number than the number of redundancy is selected as the candidate, and any random number of 1 to 9 is generated in order by the random number generation means generates to assign the random number to the storage unit of each candidate. The storage units of the number of redundancy can be selected from the one having a large value of the generated random number. In this case, the load concentrating on the newly added storage unit can be prevented.

Further, according to another method, the redundancy storing means may perform redundancy storage processing by using any method described above to the storage unit mounted on a different frame based on the table showing the operating state. In this case, the influence caused by disasters such as fire, earthquake and flood can be suppressed by setting each frame in a different place and building.

When the redundancy storing means investigates the disk residual capacity based on the table showing the operating state, one having larger residual capacity than the maximum capacity assumed in the image data stored is searched. Conversely, when the residual capacity is smaller than this value, the data indicating the filled drive is stored in the residual capacity data of the table showing the operating state. When all drives of the storage unit 1 are full, the time for investigating the disk residual capacity can be shortened by excluding the storage unit 1 from the object for selection.

Further, when the storage unit 1 or a drive judged to be unusual by the operating state inspection means exists, the control unit 2 retrieves the data storage address of the other storage unit in which the same data as the data stored in the storage unit or a drive judged to be unusual is stored based on the storage address stored in the database server 3 so as to restore the data. The data stored in the data storage address is selected and reproduced from the storage units 1 which is identified based on the table showing the operating state and is normally operated except the storage unit 1 in which the same data is stored. Thus, even if any storage unit breaks down, or is removed, the table showing the operating state to the added storage unit is automatically generated when the image data is automatically reproduced and a new storage unit is added. Thereby, maintenance operation such as the addition and removal of the storage unit can be performed without causing the system down of the power source.

The failure diagnosis means of the control unit 2 accesses the table showing the operating state periodically or irregularly. The failure diagnosis means of the control unit 2 recognizes the failure of the other control unit when the update history does not exist in between the time of access and the past predetermined time based on the update history of the table showing the operating state data due to the other control units 2. The failure diagnosis means of the control unit 2 starts one spare control unit when the spare control unit equipped.

Herein, the operating state inspection means may be operated by any one of the two control units under operation, and the failure diagnosis means may be operated by the other control unit. In this case, the flag and operation time data showing the completion of the operation of the failure diagnosis means due to the other control unit are recorded on the table showing the operating state, and one control unit checks the flag and the operation time data. Thereby, the failure of the other control unit can be also detected.

The control unit 2 may be constituted integrally with the storage unit 1 on the same substrate. In this case, for example, two storage units are set to an operation state. When one failure is discovered by the failure diagnosis means, the spare control unit can be switched to the operation state.

The update process and failure recovery process of the image database server 3 will be explained below. The image database server 3 is provided with the updating means for updating the database of the image database server 3 to the update processing of data performed through the first network N1 and for updating the database of the other image database server 3 through the second network N2.

The image database server 3 is provided with a port for updating and a port for retrieving to the load balancers 7. Both ports are opened at the normal time. Further, the updating means of one image database server 3 (DB1 in Figure) controls the last operation time of the updating means of the other image database server 3 (DB2 in Figure), and writes in and controls the time of last update on all the records of all tables.

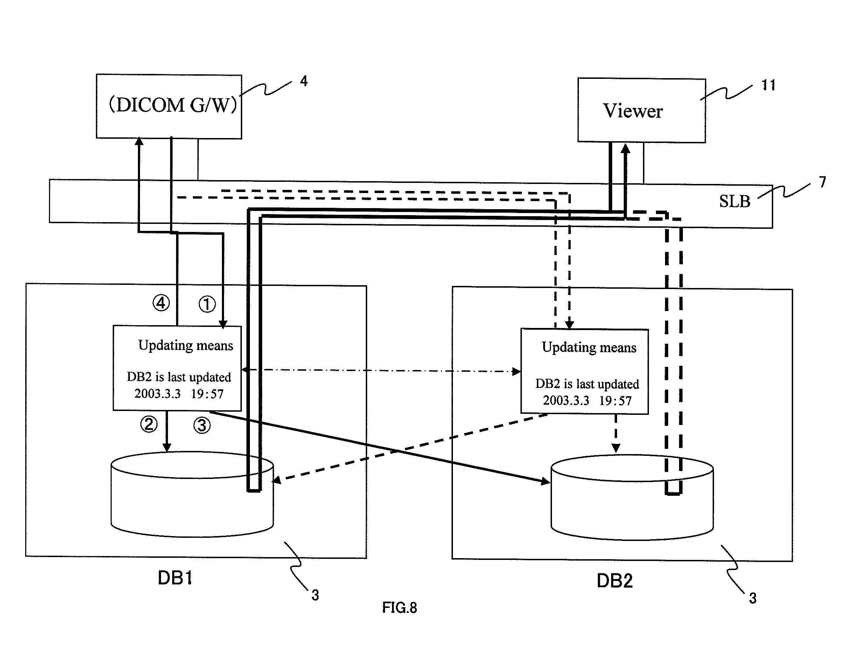

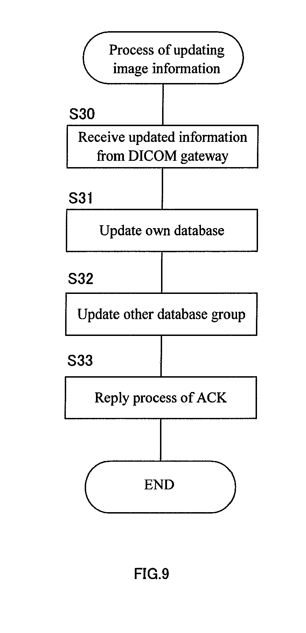

As shown in Figs. 8 and 9, the updating means updates the database (step 2 shown in Fig. 8) according to the data update request such as the storage request of the attribute information of the image data and the storage address of the image data from the DICOM gateway 4 through the load balancer 7, and the storage request of the table showing the operating state from the control unit 2, (step 1 shown in Fig. 8). Then, the updating means updates the database of the other image database server 3 through the second network 2 (step 3 shown in Fig. 8). When this operation is completed, an acknowledgement to the request is sent (step 4 shown in Fig. 8).

The image database server is provided with a port for updating data, and a port for retrieving data to the first network N 1. A recovery process means for closing the port for updating data at the time of the recovery processing to the database of the other image database server through the second network N2 is provided.

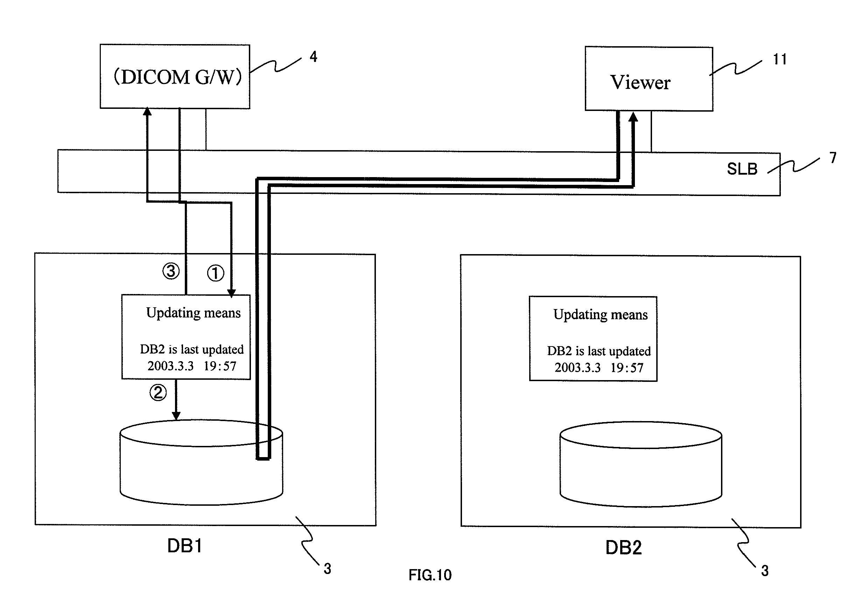

To be detailed below, when the other image database server 3 is out of order, both the port for updating data and the port for retrieving data are closed, and the load balancer 7 requires processing to one of the image database servers 3 only. As shown in Fig. 10, in this case, only the update process of own database is performed to the update request, and an acknowledgement is sent.

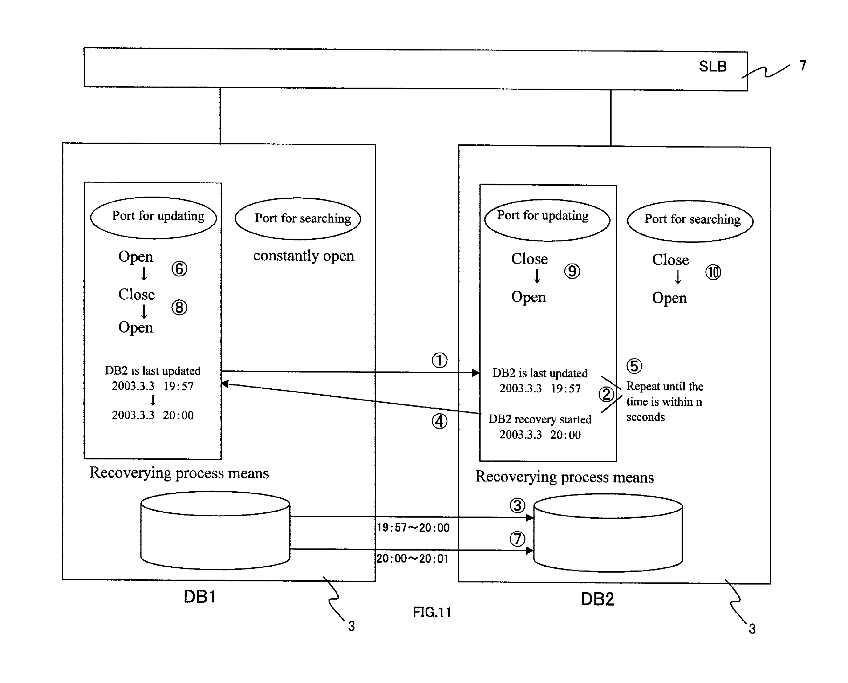

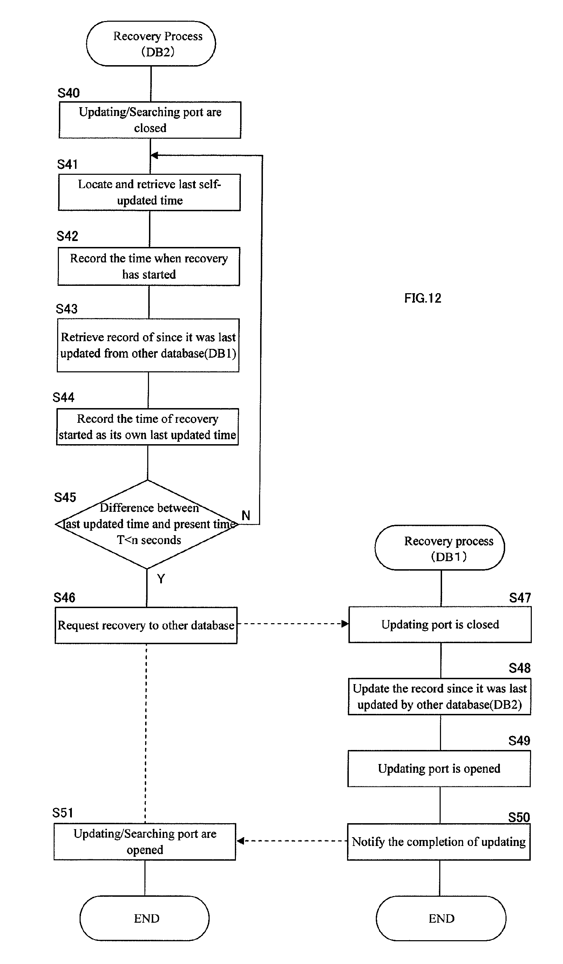

At the time of the restoration from the above failure state, one of the image database server 3 performs normal operation, and the both ports of the other image database server 3 are closed. As shown in Figs. 11 and 12, the other image database server 3 returning from failure retrieves the last self updated time from one of the image database servers 3 (step 1 shown in Fig. 11), and records restoration start time (step 2 shown in Fig. 11). Next, the record of the updated time after retrieving at the first step is retrieved from one of the image database servers 3, and the own database is updated (step 3 shown in Fig. 11). The restoration start time recorded at the step 2 is set to the last updated time of own database (step 4 shown in Fig. 11), the last updated time of the step 4 is compared with the present time. The above steps are repeated until the time is within n seconds. Herein, n is several seconds. If the time is within n seconds, the recovering operation will be requested to one of the image database servers 3 (step 5 shown in Fig. 11). On the other hand, one of the image database servers 3 closes the port for updating data, and updates the database after the time of the last update of the other image database server 3 (step 6 shown in Fig. 11) (step 7 shown in Fig. 11). The image database server 3 opens the port for updating after completing the update processing (step 8 shown in Fig. 11), and transfers the notice of termination to the other image database server 3. The other image database server 3 opens both the ports, and returns to the normal operation (steps 9, 10 shown in Fig. 11). Though both the port for updating and the port for retrieving are separately constituted in the above explanation, it is also possible to constitute the port for updating and the port for retrieving as the same port.

Another embodiment will be explained below. The above embodiment explains the system in which the high order layers of the session layer or more are operated by the DICOM protocol which is the image diagnostic standard in the medical field or the other local protocol. However, terminals such as the viewer may correspond to WEB. In this case, for example, a WEB server group containing a plurality of WEB servers is provided in the basic frame described above, and is operated under the control of the load balancer 7.

Though the above embodiments explain the storage unit constituted by the hard disk, the storage unit is not limited to the hard disk, and involatile semiconductor memories and the other media may be used.

In the above system, any one of or all of the storage unit, the control unit, the image database server 3 and the DICOM gateway is hot-swappable in the unit, and thereby the system can be maintained while the operating state of the system is maintained.

Though the above embodiments explain the image database system for storing and controlling the medical image data, it is needless to say that the system can be applied to a database system for storing and controlling the various document data other than the image.

As described above, though the suitable embodiments of the present invention are explained, the present invention is not limited thereto. Variations may be made as required without departing from the technical concept of the present invention.

### BRIEF DESCRIPTION OF THE DRAWINGS

Fig. 1 is a constitution diagram of an image database system according to the present invention;
Fig. 2 is an illustration of a procedure for storing images in the image database system according to the present invention;
Fig. 3 is a flow chart showing the procedure for storing the images in the image database system according to the present invention;
Fig. 4 is an illustration of a procedure for providing images in the image database system according to the present invention;
Fig. 5 is a flow chart showing the procedure for providing the images in the image database system according to the present invention;
Fig. 6 is an illustration of a procedure for searching images due to a viewer connected to the image database system according to the present invention;
Fig. 7 is a flow chart showing the procedure for searching the images by the viewer connected to the image database system according to the present invention;
Fig. 8 is an illustration of a data update process by an image database server.
Fig. 9 is a flow chart showing the data update process by the image database server;
Fig. 10 is an illustration of the data update process by the image database server;
Fig. 11 is an illustration of a data recovery process by the image database server;
Fig. 12 is a flow chart showing the data recovery process by the image database server; and
Fig. 13 is an illustration of a table showing an operating state.

### EXPLANATION OF REFERENCES

- 1:: Storage Unit
- 2:: Control unit
- 3:: Image database server
- 4:: image database server
- 5, 6:: Switch
- 7:: Load balancer
- 10:: Modality device
- 11:: Viewer

## Claims

1. A database system comprising:
a plurality of storage units for storing a document or image data;
a control unit for controlling the storage unit; and
a database server storing attribute information including key information associated with the document or the image data,
wherein the control unit includes:
operating state inspection means for executing an operating state inspection for investigating the data of residual capacity and the absence or presence of abnormalities to the storage unit at a predetermined timing, and storing the result on a table showing an operating state; and
redundancy storing means for selecting and storing the number of redundancy of data storage locations for data storage request from the storage units identified based on the table showing the operating state and normally operated.

2. The database system according to claim 1, wherein the redundancy storing means investigates disk residual capacity in a drive unit based on the table showing the operating state, and selects the number of redundancy of drives of which the disk residual capacity is large to the different storage unit.

3. The database system according to claim 1, wherein the redundancy storing means investigates disk residual capacity in the storage unit based on the table showing the operating state, selects the number of redundancy of drives of which the disk residual capacity is large, and selects the drive of which the disk residual capacity is large from the selected storage units.

4. The database system according to claim 1, wherein the redundancy storing means selects a plurality of storage units having residual capacity of a predetermined capacity or more as a candidate based on the table showing the operating state, and selects the storage unit as an object based on the random number generated by random number generation means.

5. The database system according to any one of claims 2 to 4, wherein the redundancy storing means selects the storage units of the number of redundancy mounted on different frames based on the table showing the operating state.

6. The database system according to any one of claims 1 to 5, wherein the data storage location is stored as the attribute information in the database server.

7. The database system according to any one of claims 1 to 6, wherein the control unit acquires the data storage locations of other storage units in which the same data as the data stored in the storage unit judged to be unusual by the operating state inspection means is stored based on the attribute information stored in the database server, selects the data stored in the data storage location from the storage units identified based on the table showing an operating state and normally operated except the storage unit in which the same data is stored, and reproduces.

8. The database system according to any one of claims 1 to 6, wherein the control unit acquires the data storage location of the other storage units in which the same data as the data stored in the drive judged to be unusual by the operating state inspection means is stored based on the attribute information stored in the database server, selects and reproduces the data stored in the data storage location from the storage units identified based on the table showing the operating state and normally operated except the drive in which the same data is stored.
